# EUROPEAN PATENT APPLICATION

(11) **EP 2 676 670 A1**
(43) Date of publication of application: **25.12.2013**
(21) Application number: 12747228.0
(22) Date of filing: 14.02.2012
(51) Int. Cl.: A61K 36/23, A23K 1/16, A23L 1/30, A23L 2/52, A61P 1/16, A61P 3/04, A61P 3/06, A61P 3/10, A61P 9/10, A61P 9/12, A61P 13/12, A61P 19/00, A61P 25/00, A61P 27/02, A61P 27/12, A61P 35/00, A61P 43/00

(54) **ADIPONECTIN PRODUCTION PROMOTER, AND MEDICINAL COMPOSITION, FOOD, DRINK AND FEED CONTAINING ADIPONECTIN PRODUCTION PROMOTER**

(30) Priority: 14.02.2011 JP 2011028932
(71) Applicant: Amino Up Chemical Co., Ltd., Sapporo-shi, Hokkaido 004-0839 (JP); Maeda, Kazuhisa, Takaishi-shi, Osaka 592-0002 (JP); Kamiyama, Yasuo, Takatsuki-shi, Osaka 569-1025 (JP); Itou, Toshinori, Toyonaka-shi, Osaka 561-0802 (JP)
(72) Inventor: MAEDA Kazuhisa, Takaishi-shi Osaka 592-0002 (JP); KAMIYAMA Yasuo, Takatsuki-shi Osaka 569-1025 (JP); ITOU Toshinori, Toyonaka-shi Osaka 561-0802 (JP)
(74) Representative: Katzameyer, Michael
(86) International application number: PCT/JP2012/053329
(87) International publication number: WO 2012/111643

(57) **Abstract**

Provided is an adiponectin production-promoting agent comprising Botanbouhu as an active component. Such an adiponectin production-promoting agent is able to be used for prevention or treatment of hypoadiponectinemia. Further provided are a pharmaceutical composition, food and drink, and feed comprising such an adiponectin production-promoting agent.

## Description

### Technical Field

The present invention relates to an adiponectin production-promoting agent, and a pharmaceutical composition, food and drink, and feed containing the adiponectin production-promoting agent.

### Background Art

Adiponectin is a kind of adipocytokines that is specifically secreted from adippcytes. It has been reported that, in association with obesity, adiponectin secretion from the adipocytes decreases. It has been also reported that the decrease in the adiponectin secretion causes arteriosclerosis, diabetes, or the like, and that adiponectin is able to serve as a biomarker for metabolic syndrome.

Adiponectin has recently drawn attention not only as the biomarker for metabolic syndrome but also as a biomarker in lifestyle-related disease type cancers or anti-aging (Non Patent Literature 1). Further, it has been reported that hypoadiponectinemia is a risk of developing cancers (Non Patent Literature 2), and that adiponectin activates longevity genes (Non Patent Literature 3).

There are some plant extracts that are thus far found and reported to have effects of promoting the adiponectin secretion. For example, Patent Literature 1 describes an adiponectin production-promoting agent having an olive leaf extract as an active component. Further, Patent Literature 2 describes an adiponectin production-promoting agent having a nutmeg extract as an active component.

Botanbouhu (*Peucedanum japonicum*) is a perennial plant belonging to the genus *Peucedanum* of the family *Apiaceae* and a native-grown plant along the coast from southern Kyushu to Okinawa. It is also called "Choumeisou (registered trademark)" or "Sakuna." In Okinawa, it has been for many years used as a food material that is effective for nutritional fortification, enhancement of liver function or the like.

It has been reported that Botanbouhu has various biologically activities. For example, Non Patent Literature 4 describes inhibitory activities against carcinogenesis; Patent Literature 3 describes activities of inhibiting disaccharidase; and Patent Literature 4 describes antioxidant actions, activities of stimulating cellular functions, and activities of inhibiting melanin production.

### Citation List

### Patent Literature

Patent Literature 1: Unexamined Japanese Patent Application Kokai Publication No. 2008-273938.
Patent Literature 2: Unexamined Japanese Patent Application Kokai Publication No. 2007-261993.
Patent Literature 3: Unexamined Japanese Patent Application Kokai Publication No. 2003-26694.
Patent Literature 4: Unexamined Japanese Patent Application Kokai Publication No. 2004-26697.

### Non Patent Literature

Non Patent Literature 1: Maeda K., Journal of Traditional Medicines Vol.28, No.1, 39-42 (2011).
Non Patent Literature 2: Yamaji T. et al, Cancer Res. 2010 Jul 1; 70(13):5430-7.
Non Patent Literature 3: Iwabu M. et al, Nature 2010 Apr 29; 464(7293):1313-9.
Non Patent Literature 4: Morioka T. et al, Cancer Letters, 2004, Vol.205, p133-141.

### Summary of Invention

### Technical Problem

However, there are no reports on adiponectin production-promoting actions in relation to Botanbouhu.

The present inventors have newly found out that Botanbouhu has an excellent adiponectin production-promoting action, thereby completing the present invention. An object of the present invention is to provide a novel adiponectin production-promoting agent that has beneficial effects, and pharmaceutical composition, food and drink, and feed comprising the adiponectin production-promoting agent.

### Solution to Problem

An adiponectin production-promoting agent according to a first viewpoint of the present invention comprises Botanbouhu as an active component.

The adiponectin production-promoting agent is able to be used for prevention or treatment of hypoadiponectinemia.

A pharmaceutical composition according to a second viewpoint of the present invention comprises the adiponectin production-promoting agent.

A food and drink according to a third viewpoint of the present invention comprises the adiponectin production-promoting agent.

A feed according to a fourth viewpoint of the present invention comprises the adiponectin production-promoting agent.

### Advantageous Effects of Invention

According to the present invention, a novel adiponectin production-promoting agent that is highly safe and has beneficial effects, and a pharmaceutical composition, food and drink, and feed comprising the adiponectin production-promoting agent is able to be provided. In particular, a novel adiponectin production-promoting agent that has beneficial effects on prevention or treatment of conditions or diseases caused by hypoadiponectinemia, and a pharmaceutical composition, food and drink, and feed comprising the adiponectin production-promoting agent is able to be provided. And, it is expected to benefit, for example, metabolic syndrome, lifestyle-related disease type cancer, anti-aging or the like.

### Brief Description of Drawings

FIG. 1A is a graph showing an adiponectin production-promoting action by Botanbouhu extract on human adipocytes when 50% ethanol is used as an extraction solvent;
FIG. 1 B is a graph showing an adiponectin production-promoting action by Botanbouhu extract on human adipocytes when water is used as an extraction solvent;
FIG. 2 is a graph showing adiponectin production-promoting action by Botanbouhu extract on human adipocytes derived from 8 subjects;
FIG. 3A is a graph showing transition of adiponectin concentration in blood serum when Botanbouhu dry powder was administered to a patient (female in her 20s) with heart failure, who was obese;
FIG. 3B is a graph showing transition of adiponectin concentration in blood serum when Botanbouhu dry powder was administered to a patient (41 year-old female) with diabetes, who was obese;
FIG. 4A is a graph showing transition of adiponectin production-promoting action by various functional food product materials in adipocytes derived from a patient (41 year-old female) with diabetes, who was severely obese;
FIG. 4B is a graph showing transition of adiponectin concentration in blood serum when Botanbouhu dry powder was administered to a patient (41 year-old female) with diabetes, who was severely obese; and
FIG. 5 is a graph showing transition of adiponectin concentration in blood serum when Botanbouhu dry powder was administered to 8 weight management outpatients.

### Description of Embodiments

Embodiments of the present invention will now be described in detail below.

An adiponectin production-promoting agent according to the present invention has Botanbouhu (*Peucedanum japonicum*) as an active component. As raw materials, Botanbouhu leaves, stems, roots, rootstocks, fruits, seeds, seed coats, flowers or the like are able to be used, wherein Botanbouhu has been cultivated for any length of period of days. Further, it is possible to use commercially available Botanbouhu extract, Botanbouhu powder or the like.

In an adiponectin production-promoting agent according to the present invention, what is able to be used is, for example, one prepared by drying Botanbouhu and powderized with a mortar or the like (hereinafter, referred to as Botanbouhu dry powder), one obtained by drying Botanbouhu as necessary and subjecting the resultant to extraction treatment (hereinafter, referred to as Botanbouhu extract) or the like.

The above extraction treatment is carried out by immersing Botanbouhu in an extraction solvent, for example, at 60°C for 30 minutes, or at room temperature for 12 hours. It is possible that Botanbouhu is dried and, for example, powderized, before subjected to the extraction treatment. Any extraction solvent is able to be used as long as it is a solvent that is usually used for extraction. For example, water, ethanol, methanol, isopropanol, acetone, 1,3-butylene glycol, ethylene glycol, propylene glycol, glycerin, acetic acid, ethyl acetate, ether, hexane or the like, or a mixture thereof is able to be used. Preferably, with consideration for effects on the human body, water, ethanol, or a mixture thereof is used. The thus obtained Botanbouhu extract is highly safe.

Solution obtained by the extraction treatment is able to be used as is as Botanbouhu extract. Or the filtrate obtained by filtering the solution or supernatant obtained by centrifuging the solution is able to be used as Botanbouhu extract. Further, the extraction solvent is able to be removed from the solution obtained by the extraction treatment, or the filtrate obtained by filtering the solution or the supernatant obtained by centrifuging the solution and the resultant is able to be used as Botanbouhu extract. Examples of filtration methods include filter filtration. Removal of the extraction solvent is carried out by, for example, drying off, while warming, the extraction solvent. It is possible that this treatment is carried out under normal pressure (atmospheric pressure) or carried out under reduced pressure.

In cases where the extraction solvent is able to be removed from the solution obtained by the extraction treatment, or the filtrate obtained by filtering the solution or the supernatant obtained by centrifuging the solution, the resultant in which the extraction solvent is removed is able to be as is as Botanbouhu extract or the resultant in which the extraction solvent is removed is able to be reconstituted in a solvent to use as Botanbouhu extract. With consideration for effects on the human body, water, ethanol, or a mixture thereof is preferably used as the solvent. The thus obtained Botanbouhu extract is highly safe. After the reconstitution, it is possible to carry out treatment such as filter sterilization.

The adiponectin production-promoting agent according to the present invention is able to promote production of adiponectin inside or outside a living organism. The adiponectin production-promoting action is able to be evaluated, for example, by orally administering Botanbouhu dry powder to a mammal or administering intravenously, intraperitoneally, or intracutaneously Botanbouhu extract to a mammal; measuring adiponectin concentration in blood serum before and after the Botanbouhu administration by ELIZA or the like; and checking if the adiponectin concentration in blood serum after the administration increases as compared with that before the administration. Further, the adiponectin production-promoting action is able to be evaluated, for example, by adding Botanbouhu extract to a medium containing adipocytes derived from mammals, culturing the cells; and checking if adiponectin concentration in the culture media increases as compared with a control medium with no Botanbouhu extract being added, or checking if adiponectin concentration in the culture medium increases with time. The method is able to be appropriately selected as long as it is a method of using adiponectin production-promoting agent that provides effects of the present invention, and a method of evaluating adiponectin production-promoting action.

It is possible that this adiponectin production-promoting action is ascribed to substances contained in Botanbouhu including, for example, chlorodiene acid, isoquercitrin, Cnidioside A, Praeroside, Apterin, Aesculin, Peusedanol, tryptophan, uracil, guanosine, uridine, thymidine, and a derivative thereof. These substances are able to be extracted and specified by, for example, a method described in HISAMOTO M at al., J. Agric. Food Chem. 2003, 51, 5255-5261. Further, the adiponectin production-promoting action of these substances is able to be evaluated, for example, by adding the substance to a medium containing adipocytes derived from mammals, culturing the cells; and checking if adiponectin concentration in the culture media increases as compared with a medium with positive control (for example, pioglitazone) being added.

The adiponectin production-promoting agent according to the present invention is able to be used for prevention or treatment of hypoadiponectinemia. Here, hypoadiponectinemia is a condition where health condition is expected to improve by promoting adiponectin secretion in the body.

Further, the adiponectin production-promoting agent according to the present invention is able to be used in prevention or treatment of conditions or diseases caused by hypoadiponectinemia. It is able to be used in prevention or treatment of, for example, metabolic syndrome, lifestyle-related disease type cancer, insulin-resistance syndrome, diabetes (including type I diabetes and type II diabetes), diabetes mellitus complications (including retinopathy, renal function disorder, neurosis, cataract, and coronary artery disease), arteriosclerosis, coronary artery disease, renal function disorder, myocardial infarction, hypertension, cerebrovascular disorder, hyperlipidemia, hypercholesterolemia, obesity, reduced bone density, hepatic disease or the like. Furthermore, the adiponectin production-promoting agent according to the present invention is able to be used for anti-aging.

The adiponectin production-promoting agent according to the present invention is able to be used in pharmaceutical compositions, food and drinks, and feed.

In cases where the adiponectin production-promoting agent according to the present invention is used in a pharmaceutical composition, a method of administering this pharmaceutical composition is able to be appropriately selected from oral administration, intravenous administration, intraperitoneal administration, intracutaneous administration, sublingual administration and the like. It is possible that this pharmaceutical composition take any dosage form. It is able to be appropriately prepared as oral solid formulations such as tablets, granules, powders, or capsules; oral liquid formulations such as internal liquid or syrup; parenteral liquid formulations such as injectable solution; or the like.

In cases where the adiponectin production-promoting agent according to the present invention is used in a pharmaceutical composition, other active components are able to be contained as appropriate. For example, preventive or therapeutic agents for hypoadiponectinemia, metabolic syndrome, lifestyle-related disease type cancer, insulin-resistance syndrome, diabetes (including type I diabetes and type II diabetes), diabetes mellitus complications (including retinopathy, renal function disorder, neurosis, cataract, and coronary artery disease), arteriosclerosis, coronary artery disease, renal function disorder, myocardial infarction, hypertension, cerebrovascular disorder, hyperlipidemia, hypercholesterolemia, obesity, reduced bone density, hepatic disease; agent for anti-aging; or the like is able to be contained.

In cases where the adiponectin production-promoting agent according to the present invention is used in a pharmaceutical composition, excipients, binders, disintegrants, thickeners, dispersing agents, reabsorption promoting agents, taste masking agents, buffers, surfactants, solubilization assisting agents, preservatives, emulsifiers, isotonic agents, stabilizers, pH adjusting agents or the like, all of which are usually used, are able to be contained as appropriate.

In cases where the adiponectin production-promoting agent according to the present invention is used in a pharmaceutical composition, the dosage amount of Botanbouhu which is an active component varies in dosage form thereof, patient's age, body weight, applicable symptoms or the like. For example, when Botanbouhu is orally administered to adults, daily dosage is preferably 5 g to 20 g in terms of Botanbouhu dry powder and 0.5 to 5.0 g in terms of Botanbouhu extract. Further, any of administration during meals, administration after meals, administration before meals, administration between meals, administration before bedtime and the like is feasible, and administration during meals or administration after meals is preferred.

In cases where the adiponectin production-promoting agent according to the present invention is used in food and drink or feed, the adiponectin production-promoting agent is able to be used in the form of granulated powder, granule, paste, gel, solid, liquid or the like.

In cases where the adiponectin production-promoting agent according to the present invention is used in food and drink or feed, excipients, binders, disintegrants, thickeners, dispersing agents, reabsorption promoting agents, taste masking agents, buffers, surfactants, solubilization assisting agents, preservatives, emulsifiers, isotonic agents, stabilizers, pH adjusting agents or the like, which are approved to be contained in the food and drink or feed, are able to be contained as appropriate.

In cases where the adiponectin production-promoting agent according to the present invention is used in food and drink, the amount of Botanbouhu taken is able to be determined with accordance to an object, form, method of utilization thereof or the like. For example, the daily amount is able to be set to preferably 5 g to 20 g in terms of Botanbouhu dry powder and 0.5 to 5 .0 g in terms of Botanbouhu extract. Further, the adiponectin production-promoting agent is able to be applied to food and drink, functional food product, food product for patients, food product for specified health use or the like that has, as a concept, prevention or improvement ofhypoadiponectinemia, metabolic syndrome, lifestyle-related disease type cancer, insulin-resistance syndrome, diabetes (including type I diabetes and type II diabetes), diabetes mellitus complications (including retinopathy, renal function disorder, neurosis, cataract, and coronary artery disease), arteriosclerosis, coronary artery disease, renal function disorder, myocardial infarction, hypertension, cerebrovascular disorder, hyperlipidemia, hypercholesterolemia, obesity, reduced bone density, hepatic disease, or agent for anti-aging and indicates that effect as necessary.

In cases where the adiponectin production-promoting agent according to the present invention is used in feed, the adiponectin production-promoting agent is able to be used in, for example, feed for mammals or the like, pet foods, supplements for pets or the like.

### Example 1

By way of examples the present invention will now be specifically described below. But the present invention is by no means limited thereto. Further, unless otherwise noted, "%" represents % by mass.

An adiponectin production-promoting action by Botanbouhu extract (sample B) with 50% ethanol being used as extraction solvent was evaluated.

### (Preparation of Botanbouhu extract (sample B) with 50% ethanol being used as an extraction solvent)

Extraction treatment was performed by allowing Botanbouhu leaves (0.5 g) to be dried to powder, allowing the resulting powder to be immersed in 20 mL of 50% ethanol at 60°C for 0.5 hours, and then stirring the resultant at room temperature for 4 hours. After the extraction treatment, filter filtration (CHROMAFIL O-20/25, filter size 0.20 µm, manufactured by Macherey-Nagel) was carried out to obtain an extract solution. This extract solution 3 mL was dried up to obtain 114.3 mg of extract. This extract was dissolved in 2 mL of dimethyl sulfoxide (DMSO). This DMSO solution was subjected to filter sterilization (MN Sterilizer PES, filter size 0.22 µm, manufactured by Macherey-Nagel), thereby obtaining sample B.

### (Evaluation of adiponectin production-promoting action)

Fat tissues were harvested from remainder tissues removed at the time of surgery of a subject (a 65 year-old male patient with colorectal cancer and complicating metabolic syndrome) who provided informed consent. These fat tissues were washed several times with PBS (phosphate buffered saline) to remove the blood vessels and then shredded. To this, 0.1% type 2 collagenase solution (manufactured by Sigma-Aldrich) 10 mL was added and incubated, while stirred well, at 37°C for one hour. The resultant was then filtered with a mesh filter (manufactured by BD Falcon) and the filtrate centrifuged for 10 minutes at 780 ×g. The pellet obtained by the centrifugation was suspended in medium for adipocyte precursor culture (PM-1, manufactured by Zen-Bio) and grown at 37°C under 5% CO₂ to confluence. In order to differentiate these cells into adipocytes, the cells were treated with adipocyte differentiation medium (DM-2, manufactured by Zen-bio) at 37°C under 5% CO₂. Eight days after the DM-2 treatment, the medium was replaced with AM-1 medium with DMSO being added, or AM-1 medium with the sample B being added (Botanbouhu extract concentration: 6.2 mg/mL) (AM-1: medium for adipocyte culture, manufactured by Zen-Bio), and, 48 hours later, adiponectin concentration in the supernatant was measured. The adiponectin concentration in the supernatant was measured using human adiponectin ELISA kit (manufactured by Otsuka Pharmaceutical Co., Ltd.) according to a protocol attached to the kit.

### (Results)

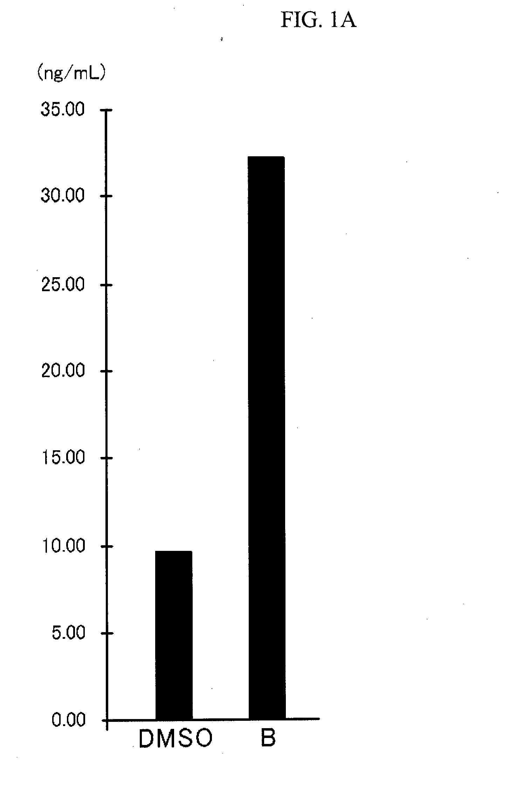

The results are shown in FIG. 1A. It was found that the adiponectin production action by adipocytes was three times or more higher in cases where the sample B was added, as compared with cases where DMSO alone was added. From this, it was revealed that Botanbouhu extract with 50% ethanol being used as an extraction solvent had an excellent adiponectin production-promoting action.

### Example 2

An adiponectin production-promoting action by Botanbouhu extract (sample A) with water being used as extraction solvent was evaluated.

### (Preparation of Botanbouhu extract (sample A) with water being used as an extraction solvent)

Extraction treatment was performed by allowing Botanbouhu leaves (0.5 g) to be dried to powder, allowing the resulting powder to be immersed in 20 mL of water at 60 °C for 0.5 hours, and then stirring the resultant at room temperature for 4 hours. After the extraction treatment, filter filtration (ADVANTEC Filter Paper Qualitative No.2, 90 mm, manufactured by ADVANTEC) was carried out to obtain 3 mL of extract solution. This extract solution was dried up to obtain 59 mg of extract. This extract was dissolved in 1 mL of DMSO. This DMSO solution was subjected to filter sterilization (MN Sterilizer PES, filter size 0.22 µm, manufactured by Macherey-Nagel), thereby obtaining sample A.

### (Evaluation of adiponectin production-promoting action)

Fat tissues were harvested from remainder tissues removed at the time of surgery of a subject (female patient in her 20s who was severely obese and had concurrently diabetes and hypoadiponectinemia) who provided informed consent. These fat tissues were treated in the same manner as in Example 1. Eight days after the DM-2 treatment, the medium was replaced with AM-1 medium with DMSO being added, or AM-1 medium with the sample A being added (Botanbouhu extract concentration: 5.6 mg/mL), and, 48 hours later, adiponectin concentration in the supernatant was measured in the same manner as in Example 1.

### (Results)

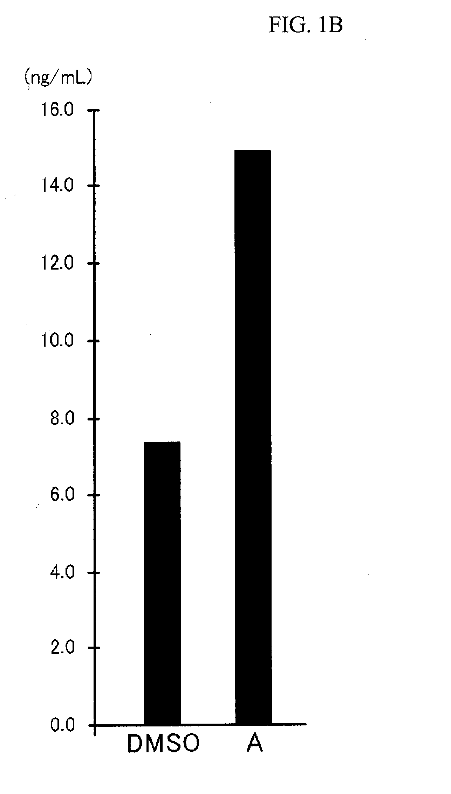

The results are shown in FIG. 1B. It was found that the adiponectin production action by adipocytes was twice or more higher in cases where the sample A was added, as compared with cases where DMSO alone was added. From this, it was revealed that, like cases where 50% ethanol was used as an extraction solvent, Botanbouhu extract with water being used as an extraction solvent had an excellent adiponectin production-promoting action.

### Example 3

In order to examine the adiponectin production-promoting action by Botanbouhu extract in more detail, the adiponectin production-promoting action was evaluated using adipocytes obtained each of 8 subjects. As the Botanbouhu extract, the sample A used in Example 2, the sample B used in Example 1 and sample C obtained by a method described below were used.

### (Preparation of Botanbouhu extract (sample C) with 100% ethanol being used as an extraction solvent)

Extraction treatment was performed by allowing Botanbouhu leaves (0.5 g) to be dried to powder, allowing the resulting powder to be immersed in 20 mL of 100% ethanol at 60°C for 0.5 hours, and then stirring the resultant at room temperature for 4 hours. After the extraction treatment, filter filtration (CHROMAFIL O-20/25, filter size 0.20 µm, manufactured by Macherey-Nagel) was carried out to obtain an extract solution. This extract solution 3 mL was dried up to obtain 24.7 mg of extract. This extract was dissolved in 1 mL of DMSO. This DMSO solution was subjected to filter sterilization (MN Sterilizer PES, filter size 0.22 µm, manufactured by Macherey-Nagel), thereby obtaining sample C.

### (Evaluation of adiponectin production-promoting action)

Fat tissues were individually harvested from remainder tissues removed at the time of surgery of each of the 8 subjects (three with severe obesity, one with liposarcoma, one with pancreatic cancer, one with prostate cancer, and two with colorectal cancer), all of whom provided informed consent. These fat tissues were treated in the same manner as in Example 1. Eight days after the DM-2 treatment, the medium was replaced with AM-1 medium with DMSO being added, AM-1 medium with the sample A being added (Botanbouhu extract concentration: 5.6 mg/mL), AM-1 medium with the sample B being added (Botanbouhu extract concentration: 6.2 mg/mL), or AM-1 medium with the sample C being added (Botanbouhu extract concentration: 1.8 mg/mL), and the adiponectin concentration in the supernatant after 48 hours (hereinafter referred to as 48 hour value) and the adiponectin concentration in the supernatant after 96 hours (hereinafter referred to as 96 hour value) were measured for the cells derived from each of the eight subjects in the same manner as in Example 1. In order to correct variability among the cells, APR (Adiponectin Production Ratio; adiponectin secretion promoting index) was calculated by dividing the 48 hour value by the 96 hour value.

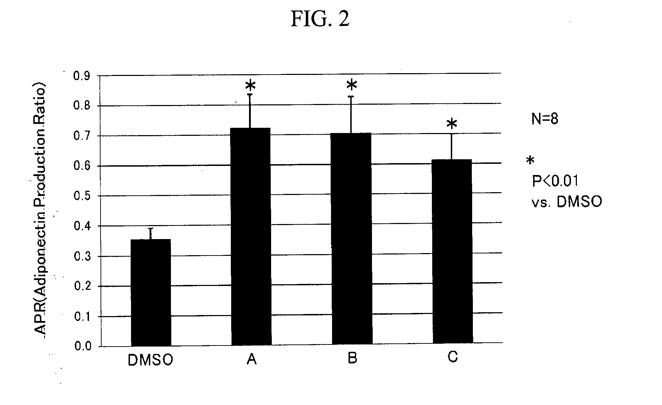

FIG. 2 shows the mean of APR calculated for the cells derived from each of 8 subjects. It was found that APR was significantly higher in any of cases where the sample A, B, or C was added, as compared with DMSO alone was added. From this, it was revealed that Botanbouhu extract had an excellent adiponectin production-promoting action.

### Example 4

An adiponectin production-promoting action in cases where Botanbouhu was actually administered orally to human was evaluated.

### (Patient with heart failure who was obese, female in her 20s)

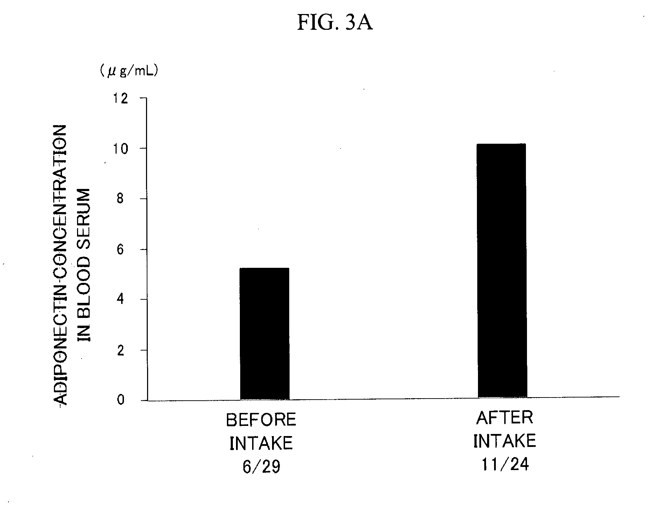

To a subject (a patient with heart failure who was obese, female in her 20s) who provided informed consent, one prepared by drying and powdering Botanbouhu leaves was administered in the amount of 10 g per day for about 5 months. The administration method thereof involved administration after meals twice a day (morning and evening). The adiponectin concentration in blood serum at the start of intake and five months after the start of intake were measured using human adiponectin ELISA kit (manufactured by Otsuka Pharmaceutical Co., Ltd.) according to a protocol attached to the kit. The results are shown in FIG. 3A. While the adiponectin concentration at the start of intake was 5.3 µg/mL, the adiponectin concentration five months after the start of intake increased to 10.1 µg/mL.

### (Patient with diabetes who was obese, 41 year-old female)

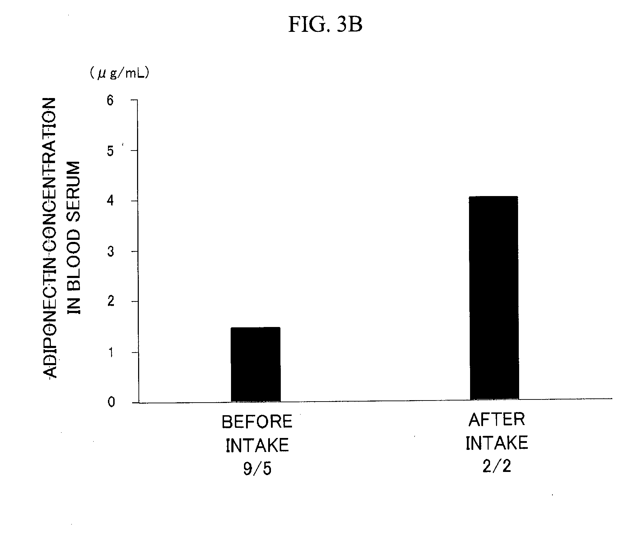

To a subject (a patient with diabetes who was obese, 41 year-old female) who provided informed consent, one prepared by drying and powdering Botanbouhu leaves was administered in the same manner as described above. The adiponectin concentration in blood serum at the start of intake and five months after the start of intake were measured in the same manner as described above. The results are shown in FIG. 3B. While the adiponectin concentration at the start of intake was 1.45 µg/mL, the adiponectin concentration five months after the start of intake increased to 4.05 µg/mL.

### (One example where Botanbouhu dry powder was administered in a tailor made manner based on information on fat stem cells)

Fat tissues were harvested from remainder tissues removed at the time of surgery of a subject (a 41 year-old female patient who was severely obese and had diabetes) who provided informed consent. These fat tissues were treated in the same manner as in Example 1 and used in screening for the adiponectin production-promoting action of functional food product materials (samples 1 to 71).

The samples 1 to 71 (Table 1 and Table 2) were suspended and dissolved in water, heated water (80°C) or DMSO, and then stirred for 30 minutes, followed by filter filtration (Φ0.45 µm) for preparation thereof. The sample 25 was prepared by filter filtration (Φ0.45 µm) without carrying out dilution. Further, with regard to the samples 1 to 18, each of the samples was made with water to 1% suspension or aqueous solution, followed by filter filtration (Φ0.45 µm) for preparation thereof. Botanbouhu extract of the samples 58 and 59 was prepared in the same manner as in Example 2. Table 1 and Table 2 show material, solvent and concentration in the culture medium of samples 1 to 71 (samples 6 to 8 "Mixture of three types of peptides" (manufactured by Katano Bussan)is a mixture of peptides derived from dried kelp or konbu, sardine and pearl oyster).

**[Table 1]**

| Sample No. | Material | Solvent | Concentration in Culture Medium | APR |
|---|---|---|---|---|
| 1 | Water | Water | - | 0.37 |
| 2 | DMSO | DMSO | 0.067% | 0.36 |
| 3 | NUCLEGEN^{®} (20%) + OLIGO RNA^{®} (80%) | Water | 0.067% | 0.43 |
| 4 | NUCLEGEN^{®} (50%) + OLIGO RNA^{®} (50%) | Water | 0.067% | 0.34 |
| 5 | NUCLEGEN^{®} (80%) + OLIGO RNA^{®} (20%) | Water | 0.067% | 0.38 |
| 6 | NUCLEGEN^{®} (20%) + Mixture of three types of peptides (80%) | Water | 0.067% | 0.39 |
| 7 | NUCLEGEN^{®} (50%) + Mixture of three types of peptides (50%) | Water | 0.067% | 0.36 |
| 8 | NUCLEGEN^{®} (20%) + Mixture of three types of peptides (80%) | Water | 0.067% | 0.31 |
| 9 | NUCLEGEN^{®} (20%) + Extract from fermenting mash of potato shochu distilled liquor (80%) | Water | 0.067% | 0.42 |
| 10 | NUCLEGEN^{®} (20%) + Extract from fermenting mash of purple potato shochu distilled liquor (80%) | Water | 0.067% | 0.37 |
| 11 | OLIGO RNA^{®} (20%) + barley young leaf extract powder (80%) | Water | 0.067% | 0.45 |
| 12 | OLIGO RNA^{®} (20%) + mulberry leaf (80%) | Water | 0.067% | 0.31 |
| 13 | OLIGO RNA^{®} (20%) + Deer Horn Shape *Ganoderma lucidum* (mulberry) (80%) | Water | 0.067% | 0.39 |
| 14 | OLIGO RNA^{®} (20%) + Deer Horn Shape *Ganoderma lucidum* (apple) (80%) | Water | 0.067% | 0.40 |
| 15 | OLIGO RNA^{®} (20%) + Jerusalem artichoke (80%) | Water | 0.067% | 0.42 |
| 16 | OLIGO RNA^{®} (20%) + silk protein (80%) | Water | 0.067% | 0.39 |
| 17 | OLIGO RNA^{®} (20%) + Extract from fermenting mash of potato shochu distilled liquor (80%) | Water | 0.067% | 0.39 |
| 18 | OLIGO RNA^{®} (20%) + Extract from fermenting mash of purple potato shochu distilled liquor (80%) | Water | 0.067% | 0.35 |
| 19 | OLIGO RNA^{®} (20%) + Tatary buckwheat (80%) | Water | 0.067% | 0.35 |
| 20 | OLIGO RNA^{®} (20%) + Fermented wheat germ extract (*Pantoea* bacteria) (80%) | Water | 0.067% | 0.12 |
| 21 | Nucleoprotein | Water | 0.067% | 0.36 |
| 22 | Watercress powder | Heated water | 0.067% | 0.36 |
| 23 | Turmeric | Water | 0.067% | 1.15 |
| 24 | Japanese plum extract | Water | 0.067% | 0.25 |
| 25 | Chaga mushroom culture solution | Water | 6.70% | 0.22 |
| 26 | Hydrangea tea | Heated water | 0.067% | 0.15 |
| 27 | Black yeast^{®} | Water | 0.067% | 0.31 |
| 28 | OLALIS^{®} | Water | 0.067% | 0.26 |
| 29 | Collagen peptide | Water | 0.067% | 0.34 |
| 30 | Mucopolysaccharide protein complex | Water | 0.067% | 0.35 |
| 31 | Hyaluronic acid | Water | 0.067% | 0.36 |
| 32 | Mix mineral yeast C^{®} | Water | 0.067% | 0.31 |
| 33 | Mix mineral yeast M^{®} | Water | 0.067% | 0.18 |
| 34 | Hyalo collagen^{®} | Water | 0.067% | 0.35 |

**[Table 2]**

| Sample No. | Material | Solvent | Concentration in Culture Medium | APR |
|---|---|---|---|---|
| 35 | Marinactive^{®} | Water | 0.067% | 0.33 |
| 36 | Fucoxanthin | Water | 0.067% | 0.34 |
| 37 | Astaxanthin | Water | 0.067% | 0.34 |
| 38 | Pomegranate bark extract powder | Water | 0.067% | 0.33 |
| 39 | *Citrus aurantium* extract powder | Water | 0.067% | 0.19 |
| 40 | Guggul extract powder | DMSO | 0.067% | 0.08 |
| 41 | DIBIA^{®} | Water | 0.067% | 0.28 |
| 42 | DOCOPEN^{®} | DMSO | 0.067% | 0.19 |
| 43 | Macademia nut oil | DMSO | 0.067% | 0.35 |
| 44 | NUCLEGEN^{®} (100%) | Water | 0.067% | 0.32 |
| 45 | OLIGO RNA^{®} (100%) | Water | 0.067% | 0.41 |
| 46 | Beer yeast blend^{®} | Water | 0.067% | 0.32 |
| 47 | Oligonol^{®}-Thick | DMSO | 20 mg/mL | 0.09 |
| 48 | Oligonol^{®}-Intermediate | DMSO | 0.2 mg/mL | 0.43 |
| 49 | Oligonol^{®}-Thin | DMSO | 0.02 mg/mL | 0.46 |
| 50 | Litchee polyphenol-Thick | DMSO | 20 mg/mL | 0.40 |
| 51 | Litchee polyphenol-Intermediate | DMSO | 0.2 mg/mL | 0.43 |
| 52 | Litchee polyphenol-Thin | DMSO | 0.02 mg/mL | 0.43 |
| 53 | AHCC^{®}-Thick | Water | 20 mg/mL | 0.42 |
| 54 | AHCC^{®}-Thin | Water | 0.02 mg/mL | 0.32 |
| 55 | GCP^{®}-Thick | DMSO | 20 mg/mL | 0.33 |
| 56 | GCP^{®}-Intermediate | DMSO | 0.2 mg/mL | 0.36 |
| 57 | GCP^{®}-Thin | DMSO | 0.02 mg/mL | 0.38 |
| 58 | Botanbouhu-Thin | Water | 0.56 mg/mL | 0.40 |
| 59 | Botanbouhu-Thick | Water | 5.6 mg/mL | 1.07 |
| 60 | Pioglitazone | Water | 10 nM | 0.96 |
| 61 | Telm isartan | Water | 10 nM | 0.54 |
| 62 | Rimonabant | Water | 10 nM | 0.69 |
| 63 | Spironolactone | Water | 10 nM | 0.60 |
| 64 | Eplerenone | Water | 10 nM | 0.62 |
| 65 | Bezafibrate | Water | 10nM | 0.75 |
| 66 | Fasudil | Water | 10 nM | 0.58 |
| 67 | Leuploid | Water | 10nM | 0.58 |
| 58 | Gliclazide | Water | 10 nM | 0.58 |
| 69 | Olmesartan | Water | 10 nM | 0.00 |
| 70 | Glimepyride | Water | 10 nM | 0.82 |
| 71 | Metformin | Water | 10 nM | 0.53 |

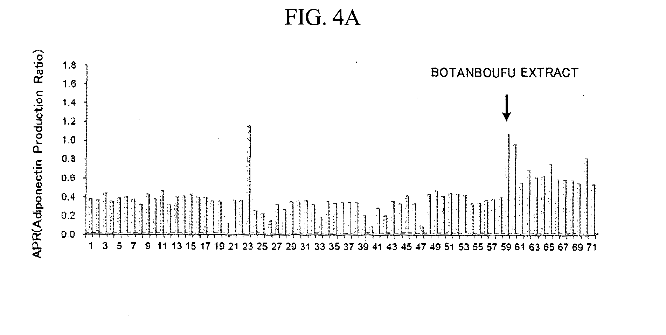

Eight days after DM-2 treatment, the medium was changed to AM-1 medium with each of the samples 1 to 71 being added and 48 hour value and 96 hour value were measured in the same manner as described in Example 3. APR (Adiponectin Production Ratio; adiponectin secretion promoting index) was calculated by dividing the 48 hour value by the 96 hour value. The results are shown in Table 1 and Table 2 and FIG. 4A. In fat stem cells from this patient, relatively higher APR value was recognized when Botanbouhu extract was added, as compared with when other functional food product materials were added (indicated by arrow in FIG. 4A).

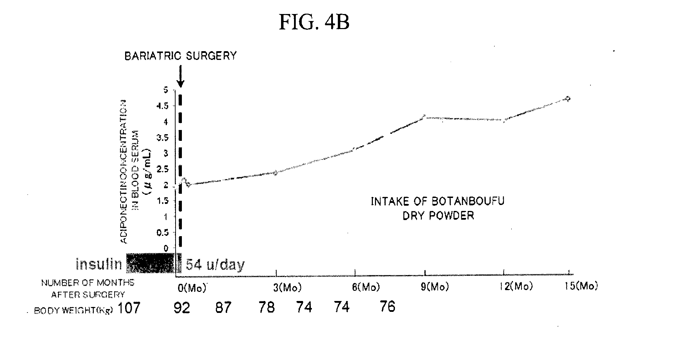

From the above, because it was predicted that adiponectin production was significantly promoted by administration of Botanbouhu in this patient, clinical evaluation was carried out. The results are shown in FIG. 4B. This patient had been administered with insulin before bariatric surgery (54 U/day). The body weight of this patient decreased by insulin administration before the surgery and further decreased after the surgery. On the other hand, adiponectin concentration in blood serum did not increase even 3 months after the surgery. For about 9.5 months starting from 4.5 months after the surgery, one prepared by drying and powderizing Botanbouhu leaves and powder was orally administered in the amount of 10 g per day (twice a day (morning and evening), administration after meals). The adiponectin concentration in blood serum significantly increased after the start of the administration of Botanbouhu dry powder (a method of measuring the concentration was same as the above).

### (Cases where administration of Botanbouhu dry powder was carried out in a weight management outpatient clinic)

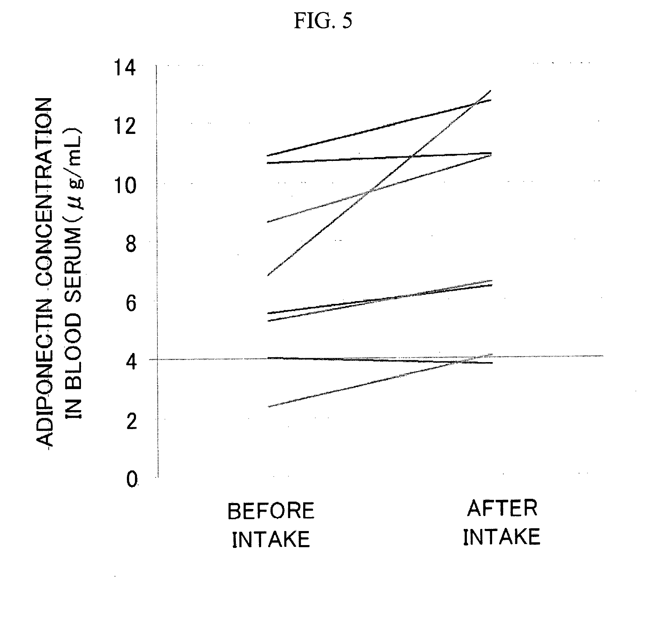

To 8 subjects (including 4 cases after bariatric surgery, average BMI: 32, 2 males, 6 females) who were weight management out patients and provided informed consent, one prepared by drying and powderizing Botanbouhu leaves and powder was orally administered in the amount of 10 g per day (twice a day (morning and evening), administration after meals). The average period of administration was 1.4 months. The results are shown in FIG. 5. In most of the cases, an increase in the adiponectin concentration in blood serum after the start of the administration of Botanbouhu dry powder was recognized (a method of measuring the concentration was same as the above).

From the above, it was recognized that adiponectin concentration in blood serum increase by administering Botanbouhu dry powder to humans. From this, it was revealed that Botanbouhu had an excellent adiponectin production-promoting action even when practically administered to humans.

As described above, Botanbouhu according to the present invention has an excellent adiponectin production-promoting action. That is, Botanbouhu according to the present invention has beneficial effects on prevention or treatment of conditions or diseases caused by hypoadiponectinemia, and is expected to have effects on, for example, metabolic syndrome, lifestyle-related disease type cancer, anti-aging or the like.

## Claims

1. An adiponectin production-promoting agent comprising:
Botanbouhu as an active component.

2. The adiponectin production-promoting agent according to Claim 1 that is used for prevention or treatment of hypoadiponectinemia.

3. A pharmaceutical composition comprising:
the adiponectin production-promoting agent according to Claim 1 or 2.

4. A food and drink comprising:
the adiponectin production-promoting agent according to Claim 1 or 2.

5. A feed comprising:
the adiponectin production-promoting agent according to Claim 1 or 2.
